(19) Europäisches Patentamt
European Patent Office
Office européen des brevets

(11) EP 2 540 299 B1

(12) **EUROPEAN PATENT SPECIFICATION**

(45) Date of publication and mention of the grant of the patent:
**13.04.2016 Bulletin 2016/15**

(21) Application number: **10846644.2**

(22) Date of filing: **16.12.2010**

(51) Int Cl.:
***A61K 31/5383*** *(2006.01)* ***A61P 27/02*** *(2006.01)*
***A61P 31/04*** *(2006.01)*

(86) International application number:
**PCT/JP2010/072685**

(87) International publication number:
**WO 2011/104981 (01.09.2011 Gazette 2011/35)**

(54) **EYE DROPS FOR TREATING EYE INFECTION CONTAINING LEVOFLOXACIN, SALT THEREOF OR SOLVATE OF SAME, METHOD FOR TREATING EYE INFECTION, LEVOFLOXACIN, SALT THEREOF OR SOLVATE OF SAME, AND UTILIZATION THEREOF**

AUGENTROPFEN ZUR BEHANDLUNG VON AUGENINFEKTIONEN MIT LEVOFLOXACIN, EINEM SALZ DARAUS ODER EINEM SOLVAT DARAUS, VERFAHREN ZUR BEHANDLUNG VON AUGENINFEKTIONEN, LEVOFLOXACIN, SALZ DARAUS ODER SOLVAT DARAUS UND VERWENDUNG

GOUTTES OPHTALMIQUES POUR LE TRAITEMENT D'UNE INFECTION OPHTALMIQUE CONTENANT DE LA LÉVOFLOXACINE, SON SEL OU SON SOLVATE, MÉTHODE DE TRAITEMENT D'UNE INFECTION OPHTALMIQUE, LÉVOFLOXACINE, SON SEL OU SON SOLVATE, ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2010 JP 2010040281**

(43) Date of publication of application:
**02.01.2013 Bulletin 2013/01**

(73) Proprietors:
- **Santen Pharmaceutical Co., Ltd**
  **Osaka-shi**
  **Osaka 533-8651 (JP)**
- **Daiichi Sankyo Company, Limited**
  **Tokyo 103-8426 (JP)**

(72) Inventors:
- **NAGANO, Takashi**
  **Ikoma-shi**
  **Nara 630-0101 (JP)**
- **SAKANAKA, Koji**
  **Ikoma-shi**
  **Nara 630-0101 (JP)**
- **NAKAMURA, Masatsugu**
  **Ikoma-shi**
  **Nara 630-0101 (JP)**
- **KAWAZU, Kouichi**
  **Ikoma-shi**
  **Nara 630-0101 (JP)**
- **IBUKI, Hajime**
  **Ikoma-shi**
  **Nara 630-0101 (JP)**
- **SAKAMOTO, Kayoko**
  **Osaka-shi**
  **Osaka 533-8651 (JP)**

(74) Representative: **Müller-Boré & Partner**
**Patentanwälte PartG mbB**
**Friedenheimer Brücke 21**
**80639 München (DE)**

(56) References cited:
**JP-A- 2005 504 818** **JP-A- 2007 526 231**
**JP-A- 2009 526 003**

- **HOLLAND EDWARD J ET AL: "The ocular penetration of levofloxacin 1.5% and gatifloxacin 0.3% ophthalmic solutions in subjects undergoing corneal transplant surgery", CURRENT MEDICAL RESEARCH AND OPINION,, vol. 23, no. 12, 1 December 2007 (2007-12-01), pages 2955-2960, XP009170074, ISSN: 1473-4877**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MCDONALD MARGUERITE B: "Research review and update: IQUIX (levofloxacin 1.5%)", INTERNATIONAL OPHTHALMOGY CLINICS, LITTLE, BROWN AND CO., BOSTON, US, vol. 46, no. 4, 1 October 2006 (2006-10-01), pages 47-60, XP009170072, ISSN: 0020-8167, DOI: 10.1097/01.IIO.0000212132.98760.72**
- **HIROSHI INOUE ET AL.: 'Levofloxacin. Cravit ophthalmic solution' CLINICS & DRUG THERAPY vol. 20, no. 4, 2001, pages 317 - 319**
- **MASAMICHI FUKUDA: 'Gan Kansensho no Nazo o Toku' GANKA PRACTICE 28. 2009, pages 276 - 277**
- **MASAMICHI FUKUDA: 'Kokin Tengan'yaku no Yakubutsu Dotai (PK) / Kokinryoku (PD) -AQCmax to Aratanaru Shihyo' IOL & RS vol. 23, no. 4, 2009, pages 531 - 536**


Remarks:

The file contains technical information submitted after the application was filed and not included in this specification

## Description

TECHNICAL FIELD

The present invention relates to an ophthalmic solution for treating an ocular infection, comprising levofloxacin or a salt thereof or a solvate of the same at a concentration of 1.5% (w/v) as an active ingredient, wherein the ophthalmic solution is used such that one drop per one eye of the ophthalmic solution is instilled three times a day. The present invention also relates to a method for treating an ocular infection by using the levofloxacin or the salt thereof or the solvate of the same. The present invention also relates to levofloxacin or a salt thereof or a solvate of the same for use in treatment of the ocular infection. Furthermore, the present invention also relates to use of the levofloxacin or the salt thereof or the solvate of the same for manufacturing an agent for treating the ocular infection.

BACKGROUND ART

Levofloxacin is one of newquinolone-based antibacterial agents that develop the antibacterial activity by inhibition of DNA gyrase and topoisomerase IV, and has been widely used. In Japan as well, because of a broad antibacterial spectrum of levofloxacin and its excellent antibacterial activity, a 0.5% (w/v) levofloxacin ophthalmic solution (Cravit® ophthalmic solution 0.5%) has been widely used as an antibacterial ophthalmic solution comprising levofloxacin as an active ingredient.

Some ocular infections cannot, however, be cured in a short time even by the 0.5% (w/v) levofloxacin ophthalmic solution. In such a case, use of the levofloxacin ophthalmic solution continues for a long time. In recent years, it has been reported that the long-term use of the 0.5% (w/v) levofloxacin ophthalmic solution leads to emergence of a resistant bacterium. For example, according to "Journal of the Eye", 21(11), 1531-1534 (2004) (NPL 1), it is reported that as a result of a bacteriological survey of conjunctival sacs of patients subjected to long-term instillation, 24% (6/25) of clinical isolates was resistant to newquinolone in a group of patients into whom levofloxacin was not instilled, whereas 71% (20/28) of clinical isolates was resistant to newquinolone in a group of patients into whom the 0.5% (w/v) levofloxacin was instilled for three months. According to "Journal of Japanese Ophthalmological Society", 110(12), 973-983 (2006) (NPL 2), it is also reported that about 10% of methicillin-sensitive Staphylococcus aureus clinically isolated from a patient suffering from bacterial keratitis was resistant to levofloxacin.

As described above, if instillation of the levofloxacin ophthalmic solution continues for a long time, there is a risk that a bacterium causing an ocular infection becomes more resistant to levofloxacin and the efficacy of levofloxacin to the ocular infection will decrease in the future. Furthermore, there is created a vicious circle that longer duration of use of the levofloxacin ophthalmic solution causes the bacterium to become more resistant to levofloxacin.

Therefore, curing the ocular infection in a short time by the levofloxacin ophthalmic solution and shortening the duration of exposure of the ocular-infection-causing bacterium to levofloxacin are very important to prevent emergence of the levofloxacin-resistant bacterium, and in turn, to ensure the future efficacy of the levofloxacin ophthalmic solution to the ocular infection. However, it is still not clear in which dosage or dose regimen the levofloxacin ophthalmic solution should be instilled to cure the ocular infection in a shorter time. In addition, change in dosage or dose regimen of the levofloxacin ophthalmic solution may result in increase in rate of occurrence of side effects as compared with the conventional dosage or dose regimen (0.5% (w/v), instilled three times a day). Therefore, when the dosage or dose regimen of the levofloxacin ophthalmic solution is changed, it is required to prevent emergence of the levofloxacin-resistant bacterium by curing the ocular infection in a shorter time than in the conventional dosage or dose regimen, and not to increase the rate of occurrence of side effects as compared with the conventional dosage or dose regimen.

PTL 1 discloses an ophthalmic solution comprising levofloxacin at a concentration of 0.3 to 4.0% (w/v) and polyalcohol. PTL 1 is, however, the invention of improving the antiseptic effect of the ophthalmic solution by blending the polyalcohol with the levofloxacin ophthalmic solution. PTL 1 neither describes nor suggests in which dosage or dose regimen the levofloxacin ophthalmic solution should be instilled to cure the ocular infection in a shorter time and to prevent emergence of the resistant bacterium.

CITATION LIST

PATENT LITERATURE

PTL 1: Japanese National Patent Publication No. 2007-526231

NON PATENT LITERATURE

NPL 1: Journal of the Eye, 21(11), 1531-1534 (2004)
NPL 2: Journal of Japanese Ophthalmological Society, 110(12), 973-983 (2006)

SUMMARY OF INVENTION

PROBLEM TO BE SOLVED BY THE INVENTION

Therefore, with respect to the levofloxacin ophthalmic solution, an interesting task is to search for the levofloxacin ophthalmic solution in a new dosage or dose regimen, which cure the ocular infection in a shorter time than in the conventional dosage or dose regimen to prevent emergence of the levofloxacin-resistant bacterium, and do not increase the rate of occurrence of side effects.

MEANS FOR SOLVING THE PROBLEM

The inventors of the present invention conducted various studies using the levofloxacin ophthalmic solution in various dosage or dose regimens. As a result of the studies, the inventors of the present invention found that bacterial conjunctivitis can be cured in a short time and the rate of occurrence of side effects does not increase in such a dosage or dose regimen that one drop per one eye of a 1.5% (w/v) levofloxacin ophthalmic solution is instilled three times a day (hereinafter also referred to as "the present dosage or dose regimen") as compared with such a dosage or dose regimen that one drop per one eye of a 0.5% (w/v) levofloxacin ophthalmic solution is instilled three times a day (hereinafter also referred to as "conventional dosage or dose regimen"), and the present invention was completed. Curing the ocular infection in a short time leads to shortening of the duration of exposure of the ocular-infection-causing bacterium to levofloxacin. Therefore, the levofloxacin ophthalmic solution in the present dosage or dose regimen is eventually expected to suppress emergence of the resistant bacterium resulting from the long-term use of the levofloxacin ophthalmic solution in the conventional dosage or dose regimen.

As a result of a study using a rabbit corneal epithelial abrasion model, the inventors of the present invention also found that although an abnormal observation is not particularly seen at the anterior eye when the 1.5% (w/v) levofloxacin ophthalmic solution is instilled three times a day, an abnormal observation and delay in curing of a corneal epithelial wound are seen at the anterior eye when a levofloxacin ophthalmic solution having a concentration of 3.0% (w/v) or higher is instilled three times a day. This finding suggests that the frequency of occurrence of side effects may increase when the levofloxacin ophthalmic solution having a concentration (dose) exceeding 1.5% (w/v) is selected.

Furthermore, the inventors of the present invention conducted a subsequent study using a bulbar conjunctiva tissue concentration simulation model. As a result of the study, the inventors of the present invention found that Staphylococcus aureus becomes resistant to levofloxacin after short-term (24-hour) use of the levofloxacin ophthalmic solution in the conventional dosage or dose regimen, whereas this is almost completely prevented surprisingly in the levofloxacin ophthalmic solution in the present dosage or dose regimen. In other words, the levofloxacin ophthalmic solution in the present dosage or dose regimen can directly inhibit the ocular-infection-causing bacterium such as Staphylococcus aureus from becoming resistant to levofloxacin, which results from the short-term use of the levofloxacin ophthalmic solution in the conventional dosage or dose regimen.

In other words, the levofloxacin ophthalmic solution in the present dosage or dose regimen is an excellent ophthalmic solution for treating an ocular infection, which treats the ocular infection in a short time without increasing side effects, and effectively inhibit an ocular-infection-causing bacterium from becoming resistant to levofloxacin, which results from the long-term and/or short-term use of levofloxacin in the conventional dosage or dose regimen.

Specifically, the present invention is directed to an ophthalmic solution for treating an ocular infection, comprising levofloxacin or a salt thereof or a solvate of the same at a concentration of 1.5% (w/v) as an active ingredient, wherein the ophthalmic solution is used such that one drop per one eye of the ophthalmic solution is instilled three times a day.

One aspect of the present invention is directed to the ophthalmic solution for treating an ocular infection, comprising levofloxacin or a salt thereof or a solvate of the same at a concentration of 1.5% (w/v) as an active ingredient, and used in the present dosage or dose regimen, wherein the ocular infection is at least one infection selected from the group consisting of conjunctivitis, blepharitis, dacryoadenitis, hordeolum, and inflammation of the tarsal gland.

Another aspect of the present invention is directed to the ophthalmic solution for treating an ocular infection, comprising levofloxacin or a salt thereof or a solvate of the same at a concentration of 1.5% (w/v) as an active ingredient, and used in the present dosage or dose regimen, wherein a bacterium causing the ocular infection is at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes.

Another aspect of the present invention is directed to the ophthalmic solution for treating conjunctivitis, comprising levofloxacin or a salt thereof or a solvate of the same at a concentration of 1.5% (w/v) as an active ingredient, and used in the present dosage or dose regimen, wherein a bacterium causing the conjunctivitis is levofloxacin-sensitive genus Staphylococcus. Preferably, the conjunctivitis is bacterial conjunctivitis, and the genus Staphylococcus is Staphylococcus aureus.

The present invention also provides a method for treating an ocular infection, comprising instilling one drop per one eye of an ophthalmic solution comprising levofloxacin or a salt thereof or a solvate of the same at a concentration of 1.5% (w/v) as an active ingredient, into a patient three times a day.

Another aspect of the present invention is directed to the method for treating an ocular infection, comprising instilling an ophthalmic solution comprising levofloxacin or a salt thereof or a solvate of the same at a concentration of 1.5% (w/v) as an active ingredient, into a patient in the present dosage or dose regimen, wherein the ocular infection is at least one infection selected from the group consisting of conjunctivitis, blepharitis, dacryoadenitis, hordeolum, and inflammation of the tarsal gland.

Another aspect of the present invention is directed to the method for treating an ocular infection, comprising instilling an ophthalmic solution comprising levofloxacin or a salt thereof or a solvate of the same at a concentration of 1.5% (w/v) as an active ingredient, into a patient in the present dosage or dose regimen, wherein a bacterium causing the ocular infection is at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes.

Another aspect of the present invention is directed to the method for treating an ocular infection, comprising instilling an ophthalmic solution comprising levofloxacin or a salt thereof or a solvate of the same at a concentration of 1.5% (w/v) as an active ingredient, into a patient in the present dosage or dose regimen, wherein a bacterium causing the ocular infection is levofloxacin-sensitive genus Staphylococcus.

Another aspect of the present invention is directed to the method for treating conjunctivitis, comprising instilling an ophthalmic solution comprising levofloxacin or a salt thereof or a solvate of the same at a concentration of 1.5% (w/v) as an active ingredient, into a patient in the present dosage or dose regimen, wherein a bacterium causing the conjunctivitis is levofloxacin-sensitive genus Staphylococcus. Preferably, the conjunctivitis is bacterial conjunctivitis, and the genus Staphylococcus is Staphylococcus aureus.

The present invention also provides levofloxacin or a salt thereof or a solvate of the same for use in treatment of an ocular infection, comprising instilling one drop per one eye of an ophthalmic solution comprising levofloxacin or a salt thereof or a solvate of the same at a concentration of 1.5% (w/v) as an active ingredient three times a day.

Another aspect of the present invention is directed to the levofloxacin or the salt thereof or the solvate of the same for use in treatment of an ocular infection, comprising instilling an ophthalmic solution comprising levofloxacin or a salt thereof or a solvate of the same at a concentration of 1.5% (w/v) as an active ingredient in the present dosage or dose regimen, wherein the ocular infection is at least one infection selected from the group consisting of conjunctivitis, blepharitis, dacryoadenitis, hordeolum, and inflammation of the tarsal gland.

Another aspect of the present invention is directed to the levofloxacin or the salt thereof or the solvate of the same for use in treatment of an ocular infection, comprising instilling an ophthalmic solution comprising levofloxacin or a salt thereof or a solvate of the same at a concentration of 1.5% (w/v) as an active ingredient in the present dosage or dose regimen, wherein a bacterium causing the ocular infection is at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes.

Another aspect of the present invention is directed to the levofloxacin or the salt thereof or the solvate of the same for use in treatment of an ocular infection, comprising instilling an ophthalmic solution comprising levofloxacin or a salt thereof or a solvate of the same at a concentration of 1.5% (w/v) as an active ingredient in the present dosage or dose regimen, wherein a bacterium causing the ocular infection is levofloxacin-sensitive genus Staphylococcus.

Another aspect of the present invention is directed to the levofloxacin or the salt thereof or the solvate of the same for use in treatment of conjunctivitis, including instilling an ophthalmic solution comprising levofloxacin or a salt thereof or a solvate of the same at a concentration of 1.5% (w/v) as an active ingredient in the present dosage or dose regimen, wherein a bacterium causing the conjunctivitis is levofloxacin-sensitive genus Staphylococcus. Preferably, the conjunctivitis is bacterial conjunctivitis, and the genus Staphylococcus is Staphylococcus aureus.

Furthermore, the present invention also provides use of levofloxacin or a salt thereof or a solvate of the same for manufacturing an ophthalmic solution for treating an ocular infection, comprising levofloxacin or a salt thereof or a

solvate of the same at a concentration of 1.5% (w/v) as an active ingredient, wherein one drop per one eye of the ophthalmic solution is instilled three times a day.

Another aspect of the present invention is directed to the use of levofloxacin or a salt thereof or a solvate of the same for manufacturing an ophthalmic solution for treating an ocular infection, comprising levofloxacin or a salt thereof or a solvate of the same at a concentration of 1.5% (w/v) as an active ingredient, and instilled in the present dosage or dose regimen, wherein the ocular infection is at least one infection selected from the group consisting of conjunctivitis, blepharitis, dacryoadenitis, hordeolum, and inflammation of the tarsal gland.

Another aspect of the present invention is directed to the use of levofloxacin or a salt thereof or a solvate of the same for manufacturing an ophthalmic solution for treating an ocular infection, comprising levofloxacin or a salt thereof or a solvate of the same at a concentration of 1.5% (w/v) as an active ingredient, and instilled in the present dosage or dose regimen, wherein a bacterium causing the ocular infection is at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes.

Another aspect of the present invention is directed to the use of levofloxacin or a salt thereof or a solvate of the same for manufacturing an ophthalmic solution for treating an ocular infection, comprising levofloxacin or a salt thereof or a solvate of the same at a concentration of 1.5% (w/v) as an active ingredient, and instilled in the present dosage or dose regimen, wherein a bacterium causing the ocular infection is levofloxacin-sensitive genus Staphylococcus.

Another aspect of the present invention is directed to the use of levofloxacin or a salt thereof or a solvate of the same for manufacturing an ophthalmic solution for treating conjunctivitis, comprising levofloxacin or a salt thereof or a solvate of the same at a concentration of 1.5% (w/v) as an active ingredient, and instilled in the present dosage or dose regimen, wherein a bacterium causing the conjunctivitis is levofloxacin-sensitive genus Staphylococcus. Preferably, the conjunctivitis is bacterial conjunctivitis, and the genus Staphylococcus is Staphylococcus aureus.

ADVANTAGEOUS EFFECTS OF INVENTION

As is clear from a result of a clinical trial described below, in the levofloxacin ophthalmic solution in the present dosage or dose regimen (1.5% (w/v), instilled three times a day), a more remarkable improvement is seen in the short-term cure rate of bacterial conjunctivitis caused by various bacteria, than in the levofloxacin ophthalmic solution in the conventional dosage or dose regimen (0.5% (w/v), instilled three times a day). In addition, although the levofloxacin ophthalmic solution in the present dosage or dose regimen has a higher concentration than that of the levofloxacin ophthalmic solution in the conventional dosage or dose regimen, the rate of occurrence of side effects does not increase. Curing the ocular infection in a short time leads to shortening of the duration of exposure of the ocular-infection-causing bacterium to levofloxacin. Therefore, the levofloxacin ophthalmic solution in the present dosage or dose regimen is eventually expected to suppress emergence of the resistant bacterium resulting from the long-term use of the levofloxacin ophthalmic solution in the conventional dosage or dose regimen.

As is also clear from a result of an ocular toxicity test described below, when the 1.5% (w/v) levofloxacin ophthalmic solution is instilled three times a day, an abnormal observation is not particularly seen at the anterior eye, whereas when the levofloxacin ophthalmic solution having a concentration of 3.0% (w/v) or higher is instilled three times a day, an abnormal observation and delay in curing of a corneal epithelial wound are seen at the anterior eye. Therefore, the frequency of occurrence of side effects may increase when the levofloxacin ophthalmic solution having a concentration (dose) exceeding 1.5% (w/v) is selected.

Furthermore, as is clear from results of an intraocular pharmacokinetics test and a pharmacological test (bulbar conjunctiva tissue concentration simulation model) described below, Staphylococcus aureus becomes resistant to levofloxacin after short-term (24-hour) use of the levofloxacin ophthalmic solution in the conventional dosage or dose regimen, whereas this is almost completely prevented surprisingly in the levofloxacin ophthalmic solution in the present dosage or dose regimen. In other words, the levofloxacin ophthalmic solution in the present dosage or dose regimen can directly inhibit the ocular-infection-causing bacterium such as Staphylococcus aureus from becoming resistant to levofloxacin, which results from the short-term use of the levofloxacin ophthalmic solution in the conventional dosage or dose regimen.

Specifically, the levofloxacin ophthalmic solution in the present dosage or dose regimen may be an excellent ophthalmic solution for treating an ocular infection, because the levofloxacin ophthalmic solution in the present dosage or dose regimen treats the ocular infection in a short time without increasing side effects, and effectively inhibit an ocular-infection-causing bacterium from becoming resistant to levofloxacin, which results from the long-term and/or short-term use of the levofloxacin ophthalmic solution in the conventional dosage or dose regimen. In addition, there are also

provided a method for treating an ocular infection by using levofloxacin or a salt thereof or a solvate of the same, levofloxacin or a salt thereof or a solvate of the same for use in treatment of the ocular infection, and use of levofloxacin or a salt thereof or a solvate of the same for manufacturing an agent for treating the ocular infection.

BRIEF DESCRIPTION OF DRAWINGS

Fig. 1 is a graph showing a change in the levofloxacin concentration in bulbar conjunctiva after a 1.5% (w/v) levofloxacin ophthalmic solution or a 0.5% (w/v) levofloxacin ophthalmic solution is instilled once, in which the vertical axis indicates the levofloxacin concentration in bulbar conjunctiva (ng/g tissue) and the horizontal axis indicates the time after administration (hr).

Fig. 2 is a graph showing a relationship between the levofloxacin concentration and the viable bacteria count for a group with 1.5% (w/v) levofloxacin pretreatment, a group with 0.5% (w/v) levofloxacin pretreatment, and a group without levofloxacin pretreatment, in which the vertical axis indicates the viable bacteria count (log CFU/mL) and the horizontal axis indicates the levofloxacin concentration (μg/mL).

DESCRIPTION OF EMBODIMENTS

Levofloxacin is a compound expressed by the following chemical structural formula (I):

[chemical formula 1]

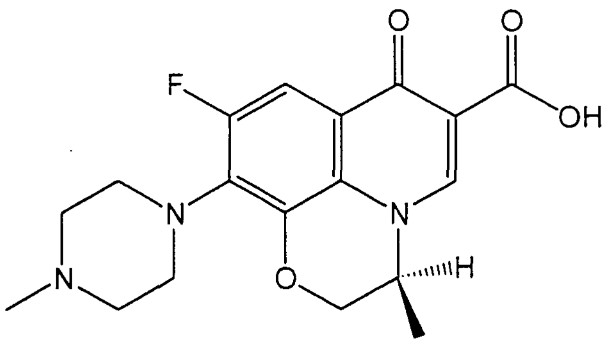

(I)

A salt of levofloxacin is not particularly limited as long as the salt is a pharmaceutically acceptable salt. Examples of the salt include: a salt with inorganic acid such as hydrochloric acid, hydrobromic acid, hydroiodic acid, nitric acid, sulfuric acid, and phosphoric acid; a salt with organic acid such as acetic acid, fumaric acid, maleic acid, succinic acid, citric acid, tartaric acid, adipic acid, gluconic acid, gluco-heptonic acid, glucuronic acid, terephthalic acid, methansulfonic acid, lactic acid, hippuric acid, 1,2-ethanedisulfonic acid, isethionic acid, lactobionic acid, oleic acid, pamo acid, polygalacturonic acid, stearic acid, tannic acid, trifluoromethansulfonic acid, benzenesulfonic acid, p-toluenesulfonic acid, laurylester sulfate, methyl sulfate, naphthalenesulfonic acid, and sulfosalicylic acid; a quaternary ammonium salt with methyl bromide, methyl iodide and the like; a salt with halogen ion such as bromine ion, chlorine ion and iodine ion; a salt with alkali metal such as lithium, sodium and potassium; a salt with alkali earth metal such as calcium and magnesium; a metal salt with iron, zinc and the like; a salt with ammonia; a salt with organic amine such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, and N,N-bis(phenylmethyl)-1,2-ethanediamine; and the like.

A solvate of levofloxacin or the salt thereof is not particularly limited as long as the solvate is a pharmaceutically acceptable solvate. Examples of the solvate include a hydrate (hemihydrate, monohydrate, dihydrate and the like), an organic solvate and the like, and the solvate is preferably a hydrate (hemihydrate, monohydrate or dihydrate).

When a crystal polymorph and a crystal polymorph group (crystal polymorph system) are present in levofloxacin or the salt thereof or the solvate of the same, these crystal polymorph and crystal polymorph group (crystal polymorph system) are also included in the scope of the present invention. The crystal polymorph group (crystal polymorph system) herein refers to individual crystal shapes in respective stages when the crystal shape changes depending on conditions and states (the states also include the formulated state) in manufacture, crystallization, storage and the like of the crystals, as well as the entire process.

Levofloxacin or the salt thereof or the solvate of the same according to the present invention is preferably a hydrate of levofloxacin, and more preferably a hemihydrate of levofloxacin.

Levofloxacin or the salt thereof or the solvate of the same can be manufactured in accordance with the methods described in Japanese Patent Publication No. 3-27534 and Japanese Patent Publication No. 7-47592. In the present invention, commercially available levofloxacin hydrochloride (manufactured by Wako Pure Chemical Industries, Ltd., catalog No.: 555-70931 and the like) can also be used.

In the present invention, a 1.5% (w/v) levofloxacin ophthalmic solution (hereinafter also referred to as "the present ophthalmic solution") refers to an ophthalmic solution comprising levofloxacin (free body) or a salt thereof or a solvate of the same at a concentration of 1.5% (w/v) as an active ingredient.

The present ophthalmic solution can be prepared by using a widely used technique and using pharmaceutically acceptable additives as necessary.

The present ophthalmic solution can be prepared by selecting from and using: for example, a tonicity agent such as sodium chloride and concentrated glycerin; a pH adjuster such as hydrochloric acid and sodium hydroxide; a buffer agent such as sodium phosphate and sodium acetate; a surfactant such as polyoxyethylene sorbitan monoolate, polyoxyl 40 stearate and polyoxyethylene hydrogenated castor oil; a stabilizing agent such as sodium citrate and sodium edetate; a preservative such as benzalkonium chloride and paraben; and the like as necessary. The present ophthalmic solution may have a pH within a range acceptable for ophthalmic drug products, and preferably have a pH of 4 to 8 usually.

In the present invention, instillation of "one drop" of the present ophthalmic solution usually refers to instillation of 10 to 60 μL of the present ophthalmic solution.

In the present invention, instillation of the present ophthalmic solution "three times a day" refers to instillation of the present ophthalmic solution three times within 24 hours, and preferably refers to instillation once in the morning, at noon and at night.

In the present invention, examples of an ocular infection include conjunctivitis, keratitis, blepharitis, dacryoadenitis, lacrimal canaliculitis, inflammation of the tarsal gland, hordeolum, endophthalmitis and the like.

In the present invention, specific examples of conjunctivitis can include bacterial conjunctivitis, catarrhal conjunctivitis, purulent conjunctivitis, pseudomembranous conjunctivitis, conjunctival phlyctenule and the like. Specific examples of keratitis can include bacterial keratitis, corneal ulcer, corneal phlyctenule and the like. Specific examples of blepharitis can include marginal blepharitis, eyelid dermatitis, angular blepharitis, staphylococcal blepharitis, seborrheic blepharitis and the like. Specific examples of dacryoadenitis can include acute dacryocystitis, chronic dacryocystitis, dacryocystitis of the newborn and the like. Specific examples of hordeolum can include external hordeolum, internal hordeolum and the like. Specific examples of endophthalmitis can include bacterial endophthalmitis, endophthalmitis after intraocular surgery and the like.

In the present invention, bacterial conjunctivitis includes acute bacterial conjunctivitis and chronic bacterial conjunctivitis, and inflammation of the tarsal gland includes meibomitis. The intraocular surgery in the present invention includes cataract surgery, glaucoma surgery, vitreoretinal surgery and the like.

The ocular infection for which the levofloxacin ophthalmic solution in the present dosage or dose regimen is used is preferably at least one infection selected from the group consisting of conjunctivitis, blepharitis, dacryoadenitis, hordeolum, and inflammation of the tarsal gland, and more preferably conjunctivitis, and further preferably bacterial conjunctivitis.

Examples of a bacterium causing the ocular infection in the present invention can include, for example, levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, Propionibacterium acnes, Neisseria gonorrhoeae, genus Bacillus, genus Clostridium, genus Comamonas and the like.

In the present invention, specific examples of the genus Staphylococcus can include Staphylococcus aureus, Coagulase Negative Staphylococcus and the like. Specific examples of the genus Streptococcus can include α-hemolytic Streptococcus, β-hemolytic Streptococcus, γ-hemolytic Streptococcus, Group A Streptococcus, Group B Streptococcus, Group C Streptococcus, Group D Streptococcus, Group E Streptococcus, Group F Streptococcus, Group G Streptococcus, Group H Streptococcus, Group K Streptococcus, Group L Streptococcus, Group M Streptococcus, Group N Streptococcus, Group O Streptococcus, Group P Streptococcus, Group Q Streptococcus, Group R Streptococcus, Group S Streptococcus, and Group T Streptococcus. Specific examples of the genus Enterococcus can include Enterococcus faecalis, Enterococcus durans and the like. Specific examples of the genus Micrococcus can include Micrococcus lylae and the like. Specific examples of the genus Moraxella can include Moraxella Branhamella catarrhalis, Moraxella-Axenfeld bacillus (including Moraxella lacunata, Moraxella liquefaciens and Moraxella bovis) and the like. Specific examples of the genus Corynebacterium can include Corynebacterium species (including Corynebacterium nebacterium diphtheriae, Corynebacterium pseudodiphthericum and Corynebacterium xerosis) and the like. Specific examples of the genus Klebsiella can include Klebsiella oxytoca, Klebsiella pneumoniae, Klebsiella terrigena, Klebsiella planticola and the like.

Specific examples of the genus Enterobacter can include Enterobacter aerogenes, Enterobacter agglomerans, Enterobacter cloacae and the like. Specific examples of the genus Serratia can include Serratia marcescens and the like. Specific examples of the genus Proteus can include Proteus mirabilis, Proteus vulgaris and the like. Specific examples of the genus Pseudomonas can include Pseudomonas alcaligenes, Pseudomonas vesicularis, Pseudomonas cepacia, Pseudomonas chlororaphis, Pseudomonas fluorescens, Pseudomonas pickettii, Pseudomonas putida and the like. Specific examples of the genus Acinetobacter can include Acinetobacter calcoaceticus, Acinetobacter baumannii, Acinetobacter lwoffii and the like. Specific examples of the genus Bacillus can include Bacillus cereus and the like. Specific examples of the genus Clostridium can include Clostridium perfringens, Clostridium tetani and the like. Specific examples of the genus Comamonas can include Comamonas acidovorans and the like.

In the present invention, the Staphylococcus aureus includes methicillin-sensitive Staphylococcus aureus (MSSA) and methicillin-resistant Staphylococcus aureus (MRSA), and the Staphylococcus epidermidis includes methicillin-sensitive Staphylococcus epidermidis (MSSE) and methicillin-resistant Staphylococcus epidermidis (MRSE). In addition, in the present invention, the Coagulase Negative Staphylococcus includes S.capitis, S.caprae, S.haemolyticus, S.hominis, S.lugdunensis, S.sciuri, S.simulans, and S.warneri, and the genus Streptococcus includes Streptococcus agalactiae, Streptococcus equisimilis, Streptococcus gordonii, Streptococcus mitis, Streptococcus morbillorum, Streptococcus oralis, and Streptococcus pyogenes.

In the present invention, "levofloxacin-sensitive" means that its strain is subjected to sufficient sterilization or bacteriostasis in levofloxacin having a clinically and usually used concentration.

The ocular infection for which the levofloxacin ophthalmic solution in the present dosage or dose regimen is used is preferably an ocular infection caused by at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes, and more preferably an ocular infection caused by levofloxacin-sensitive genus Staphylococcus, and further preferably an ocular infection caused by levofloxacin-sensitive Staphylococcus aureus, and the most preferably an ocular infection caused by levofloxacin-sensitive MSSA.

In the present invention, the ophthalmic solution for treating an ocular infection includes not only an ophthalmic solution for treating the ocular infections but also an ophthalmic solution for preventing the ocular infections. It is to be noted that prevention of the ocular infections in the present invention includes aseptic therapy in an ophthalmologic perioperative period.

The results of the clinical trial, the ocular toxicity test, the intraocular pharmacokinetics test, and the pharmacological test as well as preparation examples will be described below. These examples are for better understanding of the present invention, and do not limit the scope of the present invention.

Example

[Clinical Trial]

The clinical trial described below was conducted to compare and study influences (efficacy and safety) on bacterial conjunctivitis exerted by a group into which the 1.5% (w/v) levofloxacin ophthalmic solution was instilled three times a day in the present dosage or dose regimen and a group into which the 0.5% (w/v) levofloxacin ophthalmic solution was instilled three times a day in the conventional dosage or dose regimen.

(Method for Preparing Ophthalmic Solution)

Using the widely used technique, the 1.5% (w/v) levofloxacin ophthalmic solution and the 0.5% (w/v) levofloxacin ophthalmic solution were prepared by dissolving levofloxacin hemihydrate in water and adding a tonicity agent (glycerin) and a pH adjuster (pH: 6.1 to 6.9).

(Trial Schedule)

For up to 14 days, one drop of the 1.5% (w/v) levofloxacin ophthalmic solution or the 0.5% (w/v) levofloxacin ophthalmic solution was instilled three times a day into a patient suffering from bacterial conjunctivitis. On the starting day of instillation and 3 days, 7 days and 14 days after the starting day of instillation, which were the reference dates, the degree of eye discharge (eye secretion) and hyperemia of the patient as well as other subjective symptoms and objective observations were investigated, and a bacteria test was conducted to evaluate the number of days until the bacterium (estimated ocular-infection-causing bacterium) disappears, and the efficacy and safety of the levofloxacin

ophthalmic solution in the present dosage or dose regimen were studied.

(Method for Investigating Subjective Symptoms and Objective Observations)

An investigation on eye discharge (eye secretion) and hyperemia was conducted by scoring in accordance with Table 1 below. An investigation on other subjective symptoms and objective observations such as edema, eyelid swelling, eye pain, photophobia, and lacrimation was also conducted by scoring, in which 0 point was provided in the case where no symptom or observation was seen, and 3 points were provided in the case where the severest symptom or observation was seen (0 point, 0.5 point, 1 point, 2 points, or 3 points were provided depending on how severe the symptom or observation was).

[Table 1]

| item | judgment criteria | |
|---|---|---|
| eye discharge (eye secretion) | 0 point | no observation |
| | 0.5 point | few observations |
| | 1 point | The eye discharge is seen only at the canthus portion. |
| | 2 points | The eye discharge is seen at the palpebral conjunctiva. |
| | 3 points | The eye discharge is clearly visible to the naked eye without turning the conjunctiva out. |
| hyperemia | 0 point | no observation |
| | 0.5 point | few observations |
| | 1 point | The mild degree of vasodilatation is seen. |
| | 2 points | The moderate degree of vasodilatation is seen. |
| | 3 points | The high degree of vasodilatation is seen. |

(Method for Bacteria Test)

A specimen was taken from an infected site, and a separation identification and sensitivity test of the bacterium was conducted using a widely used method.

(Efficacy Evaluation)

The following case was judged as "short-term curing": the bacterium (estimated ocular-infection-causing bacterium) detected on the starting day of instillation (first observation day) disappeared on or before an observation day (second observation day) that was 3 days after the starting day of instillation, and a symptom judged as a main symptom by a doctor in charge, of eye discharge (eye secretion) and hyperemia, disappeared on an observation day (third observation day) that was 7 days after the starting day of instillation (the score was 0 point). However, the following case was not judged as "short-term curing": the total score of subjective symptoms and objective observations (hereinafter, collectively referred to simply as "subjective and objective symptoms") including eye discharge (eye secretion) and hyperemia as a result of the investigation by scoring on the third observation day exceeded 1/4 of the total score of subjective and objective symptoms on the starting day of instillation.

[Table 2]

| ocular-infection-causing bacterium | | group into which 1.5% levofloxacin ophthalmic solution is instilled three times a day | | group into which 0.5% levofloxacin ophthalmic solution is instilled three times a day | |
|---|---|---|---|---|---|
| | | short-term cure rate (%) | overall number of cases | short-term cure rate (%) | overall number of cases |
| Staphylococcus aureus | MSSA | 97.1 | 34 | 56.5 | 23 |
| | MRSA | 100 | 1 | 33.3 | 3 |
| Staphylococcus epidermidis | | 94.3 | 53 | 74.4 | 78 |
| Streptococcus pneumoniae | | 80.0 | 25 | 52.9 | 17 |

(continued)

| ocular-infection-causing bacterium | group into which 1.5% levofloxacin ophthalmic solution is instilled three times a day | | group into which 0.5% levofloxacin ophthalmic solution is instilled three times a day | |
|---|---|---|---|---|
| | short-term cure rate (%) | overall number of cases | short-term cure rate (%) | overall number of cases |
| Enterococcus faecalis | 100 | 4 | 83.3 | 6 |
| Corynebacterium species | 85.1 | 74 | 65.2 | 23 |
| Klebsiella oxytoca | 100 | 2 | 100 | 1 |
| Enterobacter aerogenes | 100 | 1 | 0 | 2 |
| Serratia marcescens | 100 | 1 | 66.7 | 3 |
| Proteus mirabilis | 100 | 1 | 0 | 1 |
| Comamonas acidovorans | 100 | 3 | 50.0 | 2 |
| Haemophilus influenzae | 94.1 | 17 | 85.7 | 7 |
| Propionibacterium acnes | 76.9 | 13 | 40.0 | 25 |

(Safety Evaluation)

Of occurrence or worsening of all subjective symptoms observed after administration of the levofloxacin ophthalmic solution as well as occurrence or worsening of all objective observations judged as medically harmful by the doctor in charge, those having a cause-and-effect relationship with the levofloxacin ophthalmic solution that could not be clearly denied were defined as side effects. Trialists into whom the 1.5% (w/v) levofloxacin ophthalmic solution or the 0.5% (w/v) levofloxacin ophthalmic solution was instilled once were all subject to the safety evaluation even when the trialists were not subject to the efficacy evaluation because of early dropout from the test and the like.

[Table 3]

| ocular-infection-causing bacterium | | group into which 1.5% (w/v) levofloxacin ophthalmic solution is instilled three times a day | | group into which 0.5% (w/v) levofloxacin ophthalmic solution is instilled three times a day | |
|---|---|---|---|---|---|
| | | rate of occurrence of side effects (%) | overall number of cases | rate of occurrence of side effects (%) | overall number of cases |
| Staphylococcus aureus | MSSA | 0 | 34 | 4.0 | 25 |
| | MRSA | 0 | 1 | 0 | 4 |
| Staphylococcus epidermidis | | 0 | 55 | 2.3 | 87 |
| Streptococcus pneumoniae | | 0 | 25 | 0 | 17 |
| Enterococcus faecalis | | 0 | 4 | 0 | 6 |
| Corynebacterium species | | 1.4 | 74 | 0 | 23 |
| Klebsiella oxytoca | | 0 | 2 | 0 | 1 |
| Enterobacter aerogenes | | 0 | 1 | 0 | 2 |
| Serratia marcescens | | 0 | 1 | 33.3 | 3 |
| Proteus mirabilis | | 0 | 1 | 0 | 1 |
| Comamonas acidovorans | | 0 | 3 | 0 | 2 |
| Haemophilus influenzae | | 10.5 | 19 | 0 | 7 |
| Propionibacterium acnes | | 0 | 13 | 0 | 26 |

(Test Result)

As shown in Table 2, in the group of instillation in the present dosage or dose regimen [the 1.5% (w/v) levofloxacin ophthalmic solution was instilled three times a day], a remarkable improvement was seen in the short-term cure rate for bacterial conjunctivitis, and there was obtained a high short-term cure rate of 75% or higher for all bacterial conjunctivitis caused by the tested ocular-infection-causing bacteria, as compared with the group of instillation in the conventional dosage or dose regimen [the 0.5% (w/v) levofloxacin ophthalmic solution was instilled three times a day]. Particularly when MSSA or MRSA was the ocular-infection-causing bacterium, the short-term cure rate in the present dosage or dose regimen for bacterial conjunctivitis was 97.1 % or 100%, respectively, which was significantly improved as compared with the short-term cure rate in the conventional dosage or dose regimen (56.5% or 33.3%).

Next, as shown in Table 3, no substantial difference was seen in the rate of occurrence of side effects between the group of instillation in the present dosage or dose regimen and the group of instillation in the conventional dosage or dose regimen. It is notable that the levofloxacin ophthalmic solution in the present dosage or dose regimen is equal to the levofloxacin ophthalmic solution in the conventional dosage or dose regimen in terms of the rate of occurrence of side effects although the concentration (1.5% (w/v)) of the levofloxacin ophthalmic solution in the present dosage or dose regimen is higher than the concentration (0.5% (w/v)) in the conventional dosage or dose regimen.

(Discussion)

The result of the clinical trial suggested that the levofloxacin ophthalmic solution in the present dosage or dose regimen was able to cure the ocular infections typified by bacterial conjunctivitis in a shorter time than the levofloxacin ophthalmic solution in the conventional dosage or dose regimen, and the levofloxacin ophthalmic solution in the present dosage or dose regimen was equal to the levofloxacin ophthalmic solution in the conventional dosage or dose regimen in terms of the rate of occurrence of side effects as well.

In addition, curing the ocular infection in a short time leads to shortening of the duration of exposure of the ocular-infection-causing bacterium to levofloxacin. Therefore, the levofloxacin ophthalmic solution in the present dosage or dose regimen is eventually expected to suppress emergence of the resistant bacterium resulting from the long-term use of the levofloxacin ophthalmic solution in the conventional dosage or dose regimen.

[Ocular Toxicity Test]

Using the rabbit corneal epithelial abrasion model, a study was conducted as to whether or not the levofloxacin ophthalmic solution in the present dosage or dose regimen and the levofloxacin ophthalmic solution in a dosage or dose regimen exceeding the present dosage or dose regimen caused delay in curing of a corneal epithelial wound and other abnormalities at the anterior eye.

(Method for Preparing Ophthalmic Solution)

1.5% (w/v), 3.0% (w/v) and 6.0% (w/v) levofloxacin ophthalmic solutions (pH: 6.1 to 6.9) were prepared by dissolving levofloxacin hemihydrate in water and adding a tonicity agent (glycerin) and a pH adjuster, and were used in the present test, in which an ophthalmic solution that do not contain levofloxacin was used as a vehicle.

(Method for Fabricating Rabbit Corneal Epithelial Abrasion Model)

Using male rabbits, the rabbit corneal epithelial abrasion model was fabricated in accordance with a method by Cintron et al. (Ophthalmic Res., 11, 90-96 (1979)).

(Method for Administering Drug)

The vehicle or the 1.5% (w/v), the 3.0% (w/v) or the 6.0% (w/v) levofloxacin ophthalmic solution was instilled into the rabbits three times on a day when the corneal abrasion treatment was provided as well as three times on the day following the day when the corneal abrasion treatment was provided, respectively (i.e., the ophthalmic solution was instilled three times a day for two days). The amount of the ophthalmic solution instilled at a time was 50 μL per one eye.

(Test Method)

A picture of a corneal epithelial wound site was taken immediately after corneal abrasion, and the rabbits were divided into a vehicle group as well as a 1.5% (w/v) levofloxacin instillation group, a 3.0% (w/v) levofloxacin instillation

group and a 6.0% (w/v) levofloxacin instillation group (four examples and eight eyes for each group) and the ophthalmic solution was instilled as described above. A picture of the corneal epithelial wound site was taken again 24 and 48 hours after abrasion. In addition, observations of the anterior eye (such as corneal opacity and hyperemia) were conducted before the corneal abrasion treatment and before the start of taking a picture 24 and 48 hours after abrasion. Table 4 shows symptoms at the anterior eye 24 and 48 hours after abrasion in each group. It is to be noted that the corneal epithelial wound area ratio was calculated at the respective observation times (24 and 48 hours after abrasion) in accordance with the following equation, with the corneal epithelial wound area ratio immediately after abrasion being set to 100%.

[equation 1]

corneal epithelial wound area ratio (%) = (wound area at each observation time/wound area immediately after abrasion) × 100

(Result)

Table 4 shows symptoms at the anterior eye 24 and 48 hours after abrasion in each group. The text in the parenthesis in the table indicates (the number of eyes with observation/the total number of eyes). As shown in Table 4, even when the 1.5% (w/v) levofloxacin ophthalmic solution was instilled three times a day, no abnormal observation was seen in the symptoms at the anterior eyes of the rabbits subjected to corneal abrasion. On the other hand, when the 3.0% (w/v) levofloxacin ophthalmic solution was instilled three times a day, hyperemia (8 eyes out of 8 eyes) was seen in all examples 48 hours after corneal abrasion. Furthermore, when the 6.0% (w/v) levofloxacin ophthalmic solution was instilled three times a day, hyperemia (7 eyes out of 8 eyes), eyelid swelling (4 eyes out of 8 eyes) and eye discharge (2 eyes out of 8 eyes) were seen 24 hours after corneal abrasion, and hyperemia (8 eyes out of 8 eyes) and corneal opacity (5 eyes out of 8 eyes) were seen 48 hours after abrasion.

[Table 4]

| group | 24 hours after abrasion | 48 hours after abrasion |
|---|---|---|
| vehicle group | no abnormal observation (8 eyes/8 eyes) | |
| 1.5% (w/v) levofloxacin instillation group | no abnormal observation (8 eyes/8 eyes) | |
| 3.0% (w/v) levofloxacin instillation group | no abnormal observation (8 eyes/8 eyes) | hyperemia (8 eyes/8 eyes) |
| 6.0% (w/v) levofloxacin instillation group | hyperemia (7 eyes/8 eyes)<br>eyelid swelling (4 eyes/8 eyes)<br>eye discharge (2 eyes/8 eyes) | hyperemia (8 eyes/8 eyes)<br>corneal opacity (5 eyes/8 eyes) |

In addition, as is clear from Table 5 showing the corneal epithelial wound area ratio in each group, when the 1.5% (w/v) and the 3.0% (w/v) levofloxacin ophthalmic solutions were instilled three times a day, increase in the corneal epithelial wound area ratio was not seen both 24 and 48 hours after corneal abrasion. On the other hand, when the 6.0% (w/v) levofloxacin ophthalmic solution was instilled three times a day, remarkable increase in the corneal epithelial wound area ratio was seen.

[Table 5]

| group | wound area ratio (%) | |
|---|---|---|
| | 24 hours after abrasion | 48 hours after abrasion |
| vehicle group | 54.5 | 9.5 |
| 1.5% (w/v) levofloxacin instillation group | 57.2 | 7.7 |
| 3.0% (w/v) levofloxacin instillation group | 56.7 | 7.0 |
| 6.0% (w/v) levofloxacin instillation group | 89.3 | 13.9 |

(Discussion)

Based on the above, when the 1.5% (w/v) levofloxacin ophthalmic solution was instilled three times a day, no abnormal observation and delay in curing of the corneal epithelial wound were seen at the anterior eye. On the other hand, it was suggested that when the levofloxacin ophthalmic solution having a concentration of 3.0% (w/v) or higher was instilled three times a day, an abnormal observation was seen at the anterior eye, and when the levofloxacin ophthalmic solution having a concentration of 6.0% (w/v) was instilled three times a day, delay in curing of the corneal epithelial wound was seen. Therefore, it was suggested that when the levofloxacin ophthalmic solution having a concentration (dose) exceeding 1.5% (w/v) was selected, the frequency of occurrence of side effects could increase.

[Intraocular Pharmacokinetics Test]

In order to evaluate intraocular pharmacokinetics after instillation of the 1.5% (w/v) levofloxacin ophthalmic solution and the 0.5% (w/v) levofloxacin ophthalmic solution, an intraocular pharmacokinetics test when the levofloxacin ophthalmic solution having each concentration was instilled once was conducted using the rabbits.

(Method for Preparing Ophthalmic Solution)

(1) 1.5% (w/v) levofloxacin ophthalmic solution

The 1.5% (w/v) levofloxacin ophthalmic solution was prepared using the widely used technique, by dissolving levofloxacin hemihydrate in water and adding a tonicity agent (glycerin) and a pH adjuster (pH: 6.1 to 6.9).

(2) 0.5% (w/v) levofloxacin ophthalmic solution

The commercially available Cravit® ophthalmic solution 0.5% (manufactured by Santen Pharmaceutical Co., Ltd.) was used.

(Test Method)

50 μL of the 1.5% (w/v) levofloxacin ophthalmic solution was instilled once into the right eye of the rabbit (male Japanese white rabbit, five or six examples for each group) and 50 μL of the 0.5% (w/v) levofloxacin ophthalmic solution was instilled once into the left eye. In order to remove bulbar conjunctiva, the rabbits were slaughtered 0.25, 0.5, 1, 2, 4, 6, and 8 hours after instillation, and the levofloxacin concentration in the bulbar conjunctiva was measured using a high-performance liquid chromatograph.

[Table 6]

| time after instillation (hr) | levofloxacin concentration in bulbar conjunctiva (ng/g tissue) | |
|---|---|---|
| | 1.5% (w/v) levofloxacin instillation | 0.5% (w/v) levofloxacin instillation |
| 0.25 | 14668 | 3189 |
| 0.5 | 11854 | 2486 |
| 1 | 1854 | 545 |
| 2 | 733 | 414 |
| 4 | 88 | 95 |
| 6 | 126 | 38 |
| 8 | 70 | 76 |

(Test Result and Discussion)

Table 6 and Fig. 1 show a change in the levofloxacin concentration in the bulbar conjunctiva after the 1.5% (w/v) levofloxacin ophthalmic solution or the 0.5% (w/v) levofloxacin ophthalmic solution was instilled once. Based on the test result, it was able to be confirmed that for both the 1.5% (w/v) levofloxacin ophthalmic solution and the 0.5% (w/v) levofloxacin ophthalmic solution, the concentration in the bulbar conjunctiva 8 hours after administration was

sufficiently lower than the concentration in the bulbar conjunctiva at the initial time after administration. Therefore, it was considered that the levofloxacin concentration in the bulbar conjunctiva hardly increased even when these ophthalmic solutions were instilled three times a day at eight-hour intervals.

[Pharmacological Test]

Using the bulbar conjunctiva tissue concentration simulation model, comparison and study were conducted as to whether or not the resistant bacterium emerged after the 1.5% (w/v) levofloxacin ophthalmic solution was instilled three times a day in the present dosage or dose regimen and the 0.5% (w/v) levofloxacin ophthalmic solution was instilled three times a day in the conventional dosage or dose regimen.

(Isolation of Clinical Isolates)

MSSA (MIC=0.5 μg/mL with respect to levofloxacin) was isolated from a patient suffering from an external ocular infection.

(Test Method)

The bulbar conjunctiva pharmacokinetics of levofloxacin within 24 hours after the 1.5% (w/v) levofloxacin ophthalmic solution and the 0.5% (w/v) levofloxacin ophthalmic solution were instilled three times a day were estimated from the bulbar conjunctiva pharmacokinetics after levofloxacin instillation, which was obtained in the intraocular pharmacokinetics test (Table 6 and Fig. 1). A change in the concentration thereof was re-created in an in vitro culture system (ng/g tissue of the concentration in bulbar conjunctiva was converted to μg/mL) to cause levofloxacin to act on MSSA for 24 hours. Specifically, for a time period shown in Table 7, an agar block (about 0.5 to $1 \times 10^8$ CFU/mL) containing MSSA was immersed in a Mueller Hinton broth containing levofloxacin at a concentration shown in Table 7, and the agar block was moved to the Mueller Hinton broths containing levofloxacin in a stepwise manner in the order shown in the table.

After this operation was repeated three times (after treatment with levofloxacin for a total of 24 hours), a strain was collected from the agar block and a tenfold dilution series from a $10^1$-fold dilution to a $10^6$-fold dilution were fabricated using physiological saline. 50 μL of the respective undiluted bacterial liquids and 50 μL of the respective diluted bacterial liquids were delivered by drops into Mueller Hinton agars containing 0.5, 1, 2, 4, and 8 μg/mL of levofloxacin or a Mueller Hinton agar that does not contain levofloxacin, and were allowed to stand for approximately 15 minutes. Thereafter, Conradi smearing was conducted and aerobic incubation was performed at 35°C for two days (three examples for each group). After incubation, the number of grown colonies was measured to calculate the viable bacteria count per 1 mL, and population analysis was conducted. The similar procedure was also executed in a bacterial liquid without pretreatment with levofloxacin.

[Table 7]

| | | | | | | | | | | | | | |
|---|---|---|---|---|---|---|---|---|---|---|---|---|---|
| 0.5% (w/v) levofloxacin instillation (estimated concentration in bulbar conjunctiva tissue 0 to 8 hours after instillation) | | | | | | | | | | | | | |
| action concentration (μg/mL) | 0.80 | 3.19 | 2.65 | 1.99 | 1.20 | 0.72 | 0.48 | 0.33 | 0.20 | 0.13 | 0.08 | 0.06 | |
| action time (min) | 10 | 10 | 10 | 10 | 10 | 10 | 60 | 40 | 40 | 40 | 40 | 200 | |
| 1.5% (w/v) levofloxacin instillation (estimated concentration in bulbar conjunctiva tissue 0 to 8 hours after instillation) | | | | | | | | | | | | | |
| auction concentration (μg/mL) | 3.67 | 14.7 | 12.5 | 9.12 | 4.91 | 2.65 | 1.51 | 0.95 | 0.58 | 0.34 | 0.20 | 0.12 | 0.10 |
| action time (min) | 10 | 10 | to | 10 | 10 | 10 | 30 | 30 | 30 | 30 | 30 | 30 | 240 |

(Test Result)

As is clear from Fig. 2, for MSSA pretreated with levofloxacin having a concentration corresponding to the concentration in the conjunctiva tissue when the 1.5% (w/v) levofloxacin ophthalmic solution was instilled three times a day (the present dosage or dose regimen), colonies were not formed even in a culture medium containing 0.5 μg/mL of levofloxacin and this MSSA did not become resistant to levofloxacin, similarly to the case without pretreatment with levofloxacin. On the other hand, for MSSA pretreated with levofloxacin having a concentration corresponding to the concentration in the conjunctiva tissue when the 0.5% (w/v) levofloxacin ophthalmic solution was instilled three times a day (conventional dosage or dose regimen), it was confirmed that colonies were formed even in a culture medium containing 8 μg/mL of levofloxacin and this MSSA became highly resistant to levofloxacin.

(Discussion)

Based on the result of the pharmacological test (bulbar conjunctiva tissue concentration simulation model), it was suggested that the levofloxacin ophthalmic solution in the conventional dosage or dose regimen caused MSSA to become resistant to levofloxacin 24 hours after instillation, whereas this is almost completely prevented in the levofloxacin ophthalmic solution in the present dosage or dose regimen. In other words, the levofloxacin ophthalmic solution in the present dosage or dose regimen directly inhibits the ocular-infection-causing bacterium, especially genus Staphylococcus typified by MSSA (Staphylococcus aureus), from becoming resistant to levofloxacin, which results from the short-term use of the levofloxacin ophthalmic solution in the conventional dosage or dose regimen.

Therefore, summarizing the findings obtained from the aforementioned clinical trial and the present pharmacological test (bulbar conjunctiva tissue concentration simulation model), the levofloxacin ophthalmic solution in the present dosage or dose regimen can be an excellent ophthalmic solution for treating an ocular infection, which treat the ocular infection in a short time without increasing side effects, and effectively inhibit an ocular-infection-causing bacterium from becoming resistant to levofloxacin, which results from the long-term and/or short-term use of the levofloxacin ophthalmic solution in the conventional dosage or dose regimen.

[Preparation Example]

A pharmaceutical agent according to the present invention will be described more specifically with reference to preparation examples. The present invention is not, however, limited to these preparation examples.

Formulation Example 1: ophthalmic solution (1.5% (w/v))

In 100 ml:

| | |
|---|---|
| Levofloxacin hemihydrate | 1500 mg |
| Sodium chloride | 900 mg |
| Sterile purified water | q. s. |

The aforementioned ophthalmic solution can be prepared by adding levofloxacin hemihydrate and the other aforementioned ingredients in the sterile purified water, and mixing the same sufficiently.

Formulation Example 2: ophthalmic solution (1.5% (w/v))

In 100 ml:

| | |
|---|---|
| Levofloxacin (free body) | 1500 mg |
| Concentrated glycerin | 2600 mg |
| Sterile purified water | q. s. |

The aforementioned ophthalmic solution can be prepared by adding the levofloxacin (free body) and the other aforementioned ingredients in the sterile purified water, and mixing the same sufficiently.

INDUSTRIAL APPLICABILITY

The levofloxacin ophthalmic solution in the present dosage or dose regimen has features to cure an ocular infection in a shorter time than the levofloxacin ophthalmic solution in the conventional dosage or dose regimen, and not to increase the rate of occurrence of side effects. Curing the ocular infection in a short time leads to shortening of the duration of exposure of the ocular-infection-causing bacterium to levofloxacin. Therefore, the levofloxacin ophthalmic solution in the present dosage or dose regimen is eventually expected to suppress emergence of the resistant bacterium resulting from the long-term use of the levofloxacin ophthalmic solution in the conventional dosage or dose regimen. In addition, the levofloxacin ophthalmic solution in the present dosage or dose regimen may directly inhibit the ocular-infection-causing bacterium such as Staphylococcus aureus from becoming resistant to levofloxacin, which results from the short-term use of the levofloxacin ophthalmic solution in the conventional dosage or dose regimen. In other words, the levofloxacin ophthalmic solution in the present dosage or dose regimen is an excellent ophthalmic solution for treating an ocular infection, which treat the ocular infection in a short time without increasing side effects, and effectively inhibit the ocular-infection-causing bacterium from becoming resistant to levofloxacin, which results from the long-term and/or short-term use of the levofloxacin ophthalmic solution in the conventional dosage or dose regimen.

It should be understood that the embodiments and examples disclosed herein are illustrative and not limitative in any respect. The scope of the present invention is defined by the terms of the claims, rather than the description above; and is intended to include any modifications within the scope and meaning equivalent to the terms of the claims.

## Claims

1. Levofloxacin or a salt thereof or a solvate of the same for use in treatment of an ocular infection, comprising instilling one drop per one eye of an ophthalmic solution comprising levofloxacin or a salt thereof or a solvate of the same at a concentration of 1.5% (w/v) as an active ingredient three times a day, wherein the ocular infection is at least one infection selected from the group consisting of conjunctivitis, blepharitis, dacryoadenitis, hordeolum, and inflammation of the tarsal gland.

2. The levofloxacin or the salt thereof or the solvate of the same according to claim 1, wherein the ocular infection is conjunctivitis.

3. The levofloxacin or the salt thereof or the solvate of the same according to claim 1 or 2, wherein a bacterium causing the ocular infection is at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes.

4. The levofloxacin or the salt thereof or the solvate of the same according to claim 1 or 2, wherein a bacterium causing the ocular infection is levofloxacin-sensitive genus Staphylococcus.

5. The levofloxacin or the salt thereof or the solvate of the same according to claim 1, wherein the ocular infection is conjunctivitis, and a bacterium causing the ocular infection is levofloxacin-sensitive genus Staphylococcus.

6. The levofloxacin or the salt thereof or the solvate of the same according to claim 5, wherein the conjunctivitis is bacterial conjunctivitis, and the genus Staphylococcus is Staphylococcus aureus.

7. Use of levofloxacin or a salt thereof or a solvate of the same for manufactring an ophthalmic solution for treating an ocular infection, comprising levofloxacin or a salt thereof or a solvate of the same at a concentration of 1.5% (w/v) as an active ingredient, wherein one drop per one eye of the ophthalmic solution is instilled three times a day, wherein the ocular infection is at least one infection selected from the group consisting of conjunctivitis, blepharitis, dacryoadenitis, hordeolum, and inflammation of the tarsal gland.

8. The use according to claim 7, wherein the ocular infection is conjunctivitis.

9. The use according to claim 7 or 8, wherein a bacterium causing the ocular infection is at least one type of bacterium selected from the group consisting of levofloxacin-sensitive genus Staphylococcus, genus Streptococcus, Streptococcus pneumoniae, genus Enterococcus, genus Micrococcus, genus Moraxella, genus Corynebacterium, genus

Klebsiella, genus Enterobacter, genus Serratia, genus Proteus, Morganella morganii, Haemophilus influenzae, Haemophilus aegyptius, genus Pseudomonas, Pseudomonas aeruginosa, Stenotrophomonas maltophilia, genus Acinetobacter, and Propionibacterium acnes.

**10.** The use according to claim 7 or 8, wherein a bacterium causing the ocular infection is levofloxacin-sensitive genus Staphylococcus.

**11.** The use according to claim 7, wherein the ocular infection is conjunctivitis, and a bacterium causing the ocular infection is levofloxacin-sensitive genus Staphylococcus.

**12.** The use according to claim 11, wherein the conjunctivitis is bacterial conjunctivitis, and the genus Staphylococcus is Staphylococcus aureus.

## Patentansprüche

**1.** Levofloxacin oder ein Salz davon oder ein Solvat derselben zur Verwendung in der Behandlung einer okularen Infektion, umfassend das drei Mal tägliche Verabreichen eines Tropfens pro einem Auge von einer ophthalmologischen Lösung, umfassend Levofloxacin oder ein Salz davon oder ein Solvat derselben in einer Konzentration von 1,5% (w/v) als einen aktiven Inhaltsstoff, wobei die okulare Infektion mindestens eine Infektion, ausgewählt aus der Gruppe, bestehend aus Konjunktivitis, Blepharitis, Dakryoadenitis, Hordeolum und Entzündung der Tarsaldrüse, ist.

**2.** Levofloxacin oder ein Salz davon oder ein Solvat derselben nach Anspruch 1, wobei die okulare Infektion Konjunktivitis ist.

**3.** Levofloxacin oder ein Salz davon oder ein Solvat derselben nach Anspruch 1 oder 2, wobei ein Bakterium, welches die okulare Infektion verursacht, mindestens ein Typ von Bakterium, ausgewählt aus der Gruppe, bestehend aus Levofloxacin-sensitives Genus Staphylococcus, Genus Streptococcus, Streptococcus pneumoniae, Genus Enterococcus, Genus Micrococcus, Genus Moraxella, Genus Corynebacterium, Genus Klebsiella, Genus Enterobacter, Genus Serratia, Genus Proteus, Morganella morganii, Haemophilus influenzae, Haemophilus aegyptius, Genus Pseudomonas, Pseudomonas aeruginosa, Stenotrophomonas maltophilia, Genus Acinetobacter und Propionibacterium acnes, ist.

**4.** Levofloxacin oder ein Salz davon oder ein Solvat derselben nach Anspruch 1 oder 2, wobei ein Bakterium, welches die okulare Infektion verursacht, Levofloxacin-sensitives Genus Staphylococcus ist.

**5.** Levofloxacin oder ein Salz davon oder ein Solvat derselben nach Anspruch 1, wobei die okulare Infektion Konjunktivitis ist und ein Bakterium, welches die okulare Infektion verursacht, Levofloxacin-sensitives Genus Staphylococcus ist.

**6.** Levofloxacin oder ein Salz davon oder ein Solvat derselben nach Anspruch 5, wobei die Konjunktivitis bakterielle Konjunktivitis ist und das Genus Staphylococcus Staphylococcus aureus ist.

**7.** Verwendung von Levofloxacin oder einem Salz davon oder eines Solvats derselben zur Herstellung einer ophthalmologischen Lösung zur Behandlung einer okularen Infektion, umfassend Levofloxacin oder ein Salz davon oder ein Solvat derselben in einer Konzentration von 1,5% (w/v) als einen aktiven Inhaltsstoff, wobei ein Tropfen der ophthalmologischen Lösung pro einem Auge drei Mal täglich verabreicht wird, wobei die okulare Infektion mindestens eine Infektion, ausgewählt aus der Gruppe, bestehend aus Konjunktivitis, Blepharitis, Dakryoadenitis, Hordeolum und Entzündung der Tarsaldrüse, ist.

**8.** Verwendung nach Anspruch 7, wobei die okulare Infektion Konjunktivitis ist.

**9.** Verwendung nach Anspruch 7 oder 8, wobei ein Bakterium, welches die okulare Infektion verursacht, mindestens ein Typ von Bakterium, ausgewählt aus der Gruppe, bestehend aus Levofloxacin-sensitives Genus Staphylococcus, Genus Streptococcus, Streptococcus pneumoniae, Genus Enterococcus, Genus Micrococcus, Genus Moraxella, Genus Corynebacterium, Genus Klebsiella, Genus Enterobacter, Genus Serratia, Genus Proteus, Morganella morganii, Haemophilus influenzae, Haemophilus aegyptius, Genus Pseudomonas, Pseudomonas aeruginosa, Stenotrophomonas maltophilia, Genus Acinetobacter und Propionibacterium acnes, ist.

**10.** Verwendung nach Anspruch 7 oder 8, wobei ein Bakterium, welches die okulare Infektion verursacht, Levofloxacin-sensitives Genus Staphylococcus ist.

**11.** Verwendung nach Anspruch 7, wobei die okulare Infektion Konjunktivitis ist und ein Bakterium, welches die okulare Infektion verursacht, Levofloxacin-sensitives Genus Staphylococcus ist.

**12.** Verwendung nach Anspruch 11, wobei die Konjunktivitis bakterielle Konjunktivitis ist und das Genus Staphylococcus Staphylococcus aureus ist.

**Revendications**

**1.** Lévofloxacine ou l'un de ses sels ou solvates pour une utilisation dans le traitement d'une infection oculaire, comprenant l'instillation d'une goutte par oeil d'une solution ophtalmique comprenant de la lévofloxaxine ou l'un de ses sels ou solvates à une concentration de 1,5 % (p/v) en tant que principe actif trois fois par jour, l'infection oculaire étant au moins une affection choisie dans le groupe constitué de conjonctivite, blépharite, dacryoadénite, orgelet, et inflammation des glandes de Meibomius.

**2.** Lévofloxacine ou l'un de ses sels ou solvates selon la revendication 1, où l'infection oculaire est une conjonctivite.

**3.** Lévofloxacine ou l'un de ses sels ou solvates selon la revendication 1 ou 2, où une bactérie provoquant l'infection oculaire est au moins un type de bactérie choisi dans le groupe constitué du genre Staphylococcus sensible à la lévofloxacine, du genre Streptococcus, Streptococcus pneumoniae, du genre Enterococcus, du genre Micrococcus, du genre Moraxella, du genre Corynebacterium, du genre Klebsiella, du genre Enterobacter, du genre Serratia, du genre Proteus, Morganella morganii, Haemophilus influenzae, Haemophilus aegyptius, du genre Pseudomonas, Pseudomonas aeruginosa, Stenotrophomonas maltophilia, du genre Acinetobacter, et Propionibacterium acnes.

**4.** Lévofloxacine ou l'un de ses sels ou solvates selon la revendication 1 ou 2, où une bactérie provoquant l'infection oculaire est du genre Staphylococcus sensible à la lévofloxacine.

**5.** Lévofloxacine ou l'un de ses sels ou solvates selon la revendication 1, où l'infection oculaire est une conjonctivite, et une bactérie provoquant l'infection oculaire est du genre Staphylococcus sensible à la lévofloxacine.

**6.** Lévofloxacine ou l'un de ses sels ou solvates selon la revendication 5, où la conjonctivite est une conjonctivite bactérienne, et le genre Staphylococcus est Staphylococcus aureus.

**7.** Utilisation de lévofloxacine ou de l'un de ses sels ou solvates pour la fabrication d'une solution ophtalmique pour le traitement d'une infection oculaire, comprenant de la lévofloxacine ou l'un de ses sels ou solvates à une concentration de 1,5 % (p/v) en tant que principe actif, dans laquelle une goutte par oeil de la solution ophtalmique est instillée trois fois par jour, dans laquelle l'infection oculaire est au moins une infection choisie dans le groupe constitué par la conjonctivite, la blépharite, la dacryoadénite, l'orgelet, et l'inflammation des glandes de Meibomius.

**8.** Utilisation selon la revendication 7, dans laquelle l'infection oculaire est une conjonctivite.

**9.** Utilisation selon la revendication 7 ou 8, dans laquelle une bactérie provoquant l'infection oculaire est au moins un type de bactérie choisi dans le groupe constitué du genre Staphylococcus sensible à la lévofloxacine, du genre Streptococcus, Streptococcus pneumoniae, du genre Enterococcus, du genre Micrococcus, du genre Moraxella, du genre Corynebacterium, du genre Klebsiella, du genre Enterobacter, du genre Serratia, du genre Proteus, Morganella morganii, Haemophilus influenzae, Haemophilus aegyptius, du genre Pseudomonas, Pseudomonas aeruginosa, Stenotrophomonas maltophilia, du genre Acinetobacter, et Propionibacterium acnes.

**10.** Utilisation selon la revendication 7 ou 8, dans laquelle une bactérie provoquant l'infection oculaire est du genre Staphylococcus sensible à la lévofloxacine.

**11.** Utilisation selon la revendication 7, dans laquelle l'infection oculaire est une conjonctivite, et une bactérie provoquant l'infection oculaire est du genre Staphylococcus sensible à la lévofloxacine.

**12.** Utilisation selon la revendication 11, dans laquelle la conjonctivite est une conjonctivite bactérienne, et le genre

Staphylococcus est Staphylococcus aureus.

FIG.1

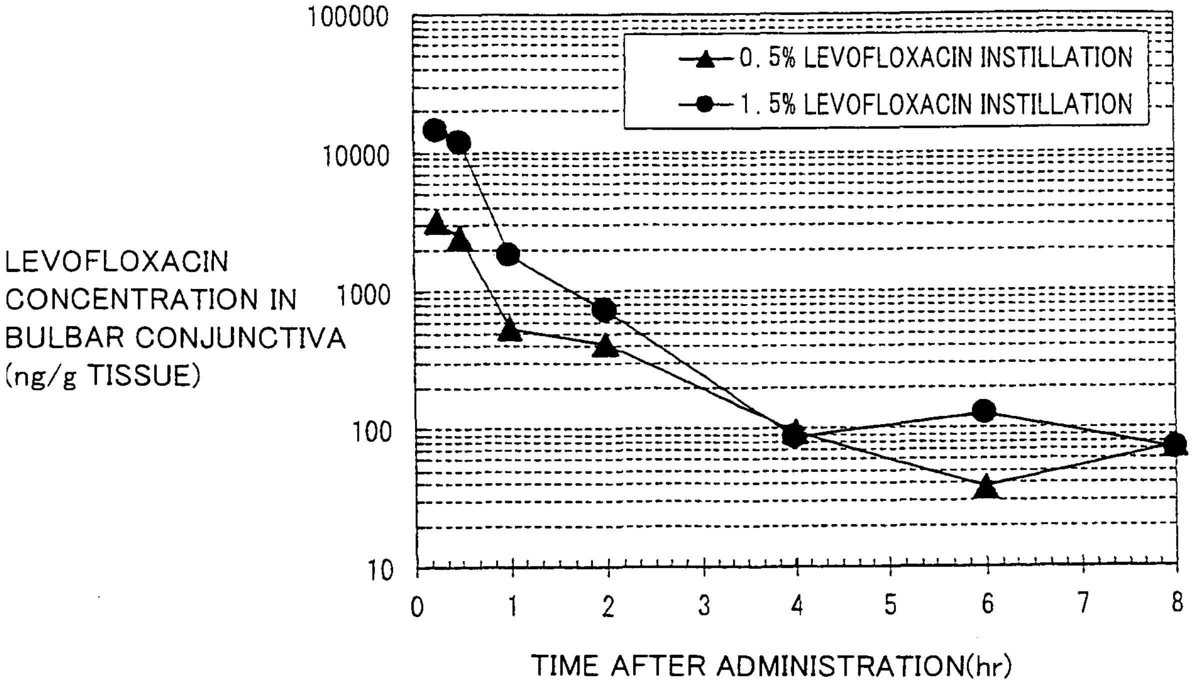
100000
10000
1000
100
10
LEVOFLOXACIN CONCENTRATION IN BULBAR CONJUNCTIVA (ng/g TISSUE)
0. 5% LEVOFLOXACIN INSTILLATION
1. 5% LEVOFLOXACIN INSTILLATION
0
1
2
3
4
5
6
7
8
TIME AFTER ADMINISTRATION(hr)

FIG.2

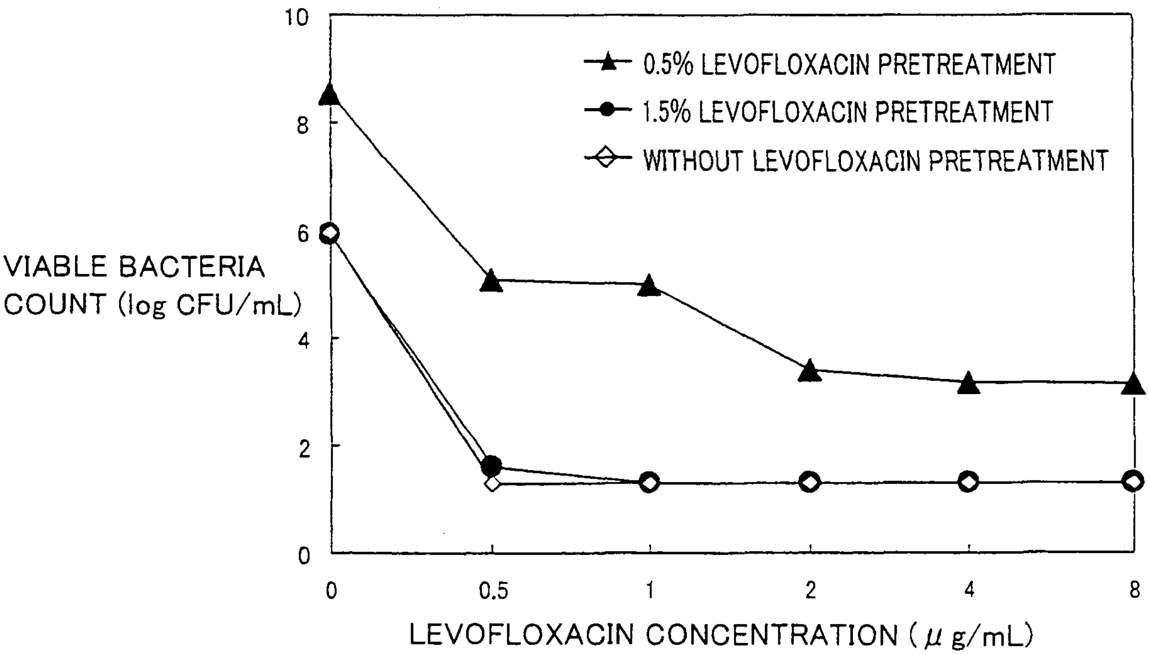
10
8
6
4
2
0
VIABLE BACTERIA COUNT (log CFU/mL)
0.5% LEVOFLOXACIN PRETREATMENT
1.5% LEVOFLOXACIN PRETREATMENT
WITHOUT LEVOFLOXACIN PRETREATMENT
0
0.5
1
2
4
8
LEVOFLOXACIN CONCENTRATION (μg/mL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007526231 A
- JP 3027534 A
- JP 7047592 A


### Non-patent literature cited in the description


- *Journal of the Eye,* 2004, vol. 21 (11), 1531-1534
- *Journal of Japanese Ophthalmological Society,* 2006, vol. 110 (12), 973-983
- **CINTRON et al.** *Ophthalmic Res.,* 1979, vol. 11, 90-96